(19) Europäisches Patentamt
European Patent Office
Office européen des brevets

(11) Veröffentlichungsnummer : **0 034 750**
**B1**

(12) # EUROPÄISCHE PATENTSCHRIFT

(45) Veröffentlichungstag der Patentschrift : **18.01.84**

(51) Int. Cl.³ : **C 07 C125/067**

(21) Anmeldenummer : **81100921.6**

(22) Anmeldetag : **10.02.81**

(54) **N-substituierte Imido-dicarbonsäure-diarylester und Verfahren zu ihrer Herstellung.**

(30) Priorität : **20.02.80 DE 3006226**

(43) Veröffentlichungstag der Anmeldung : **02.09.81 Patentblatt 81/35**

(45) Bekanntmachung des Hinweises auf die Patenterteilung : **18.01.84 Patentblatt 84/03**

(84) *Benannte Vertragsstaaten* : **BE CH DE FR GB IT LI NL**

(56) Entgegenhaltungen :
**DE-A- 2 132 936**
**JOURNAL OF THE AMERICAN CHEMICAL SOCIETY Band 69, November 1947 TOMPKINS et al. "Derivatives of Urethan; Azamalonic Esters" Seiten 2616 bis 2618**

(73) Patentinhaber : **BAYER AG**
**Zentralbereich Patente, Marken und Lizenzen**
**D-5090 Leverkusen 1, Bayerwerk (DE)**

(72) Erfinder : **Dickoré, Karlfried, Dr.**
**Nicolai-Hartmann-Strasse 9**
**D-5090 Leverkusen (DE)**
Erfinder : **Kühle, Engelbert, Dr.**
**von Bodelschwinghstrasse 42**
**D-5060 Berg.-Gladbach 2 (DE)**

**EP 0 034 750 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

N-substituierte Imido-dicarbonsäure-diarylester und Verfahren zu ihrer Herstellung

Die vorliegende Erfindung betrifft ein neues Verfahren zur Herstellung von N-substituierten Imidodicarbonsäurediarylestern, sowie bestimmte neue Verbindungen dieser Gruppe, welche als Zwischenprodukte für die Synthese von Herbiziden verwendet werden können.

Bisher sind als einzige Vertreter aus der Gruppe der N-substituierten Imido-dicarbonsäure-diarylester nur einige N-Methyl-imido-dicarbonsäure-diarylester bekannt ; diese besitzen insektizide und akarizide Eigenschaften. Sie können hergestellt werden, indem man N-Chlorcarbonyl-N-methylcarbamidsäurearylester mit Phenolen in Gegenwart eines Verdünnungsmittels und gegebenenfalls in Gegenwart eines Säurebindemittels bei Temperaturen zwischen 0 und 100 °C, vorzugsweise zwischen 20 und 40 °C, umsetzt (vgl. DE-A-2 132 936). Dieses Verfahren hat jedoch den technischen Nachteil, daß die für die Herstellung von homologen N-Alkylderivaten als Ausgangsstoffe benötigten N-Chlorcarbonyl-N-alkylcarbamidsäurearylester zum Teil nicht bekannt und in jedem Falle nur durch außerordentlich aufwendige, mehrstufige Verfahren und mit mäßigen Ausbeuten zugänglich sind. Dies gilt insbesondere für Ausgangsstoffe mit höheren, verzweigten N-Alkylgruppen, so daß das genannte Verfahren in der praktischen Anwendbarkeit im wesentlichen auf die Synthese von N-Methylderivaten beschränkt ist.

Bekannt ist weiterhin ein Verfahren zur Herstellung von N-substituierten Imido-dicarbonsäuredialkylestern durch Umsetzung von N-substituierten Carbamidsäurealkylestern mit Kohlensäure-alkylester-chloriden in Gegenwart von metallischem Natrium (vgl. J. Amer. Chem. Soc. *69,* 2616-2618 (1947)). Wie weiter unten näher erläutert, ist es jedoch unerwarteterweise nicht möglich, dieses Verfahren auf die Herstellung entsprechender O,O-Diarylester zu übertragen.

Für die Herstellung von N-substituierten Imido-dicarbonsäure-diarylestern mit höheren, insbesondere auch verzweigten N-Alkylresten stand somit kein befriedigendes, technisch brauchbares Verfahren zur Verfügung.

Es wurde nun überraschend gefunden, daß man die N-substituierten Imido-dicarbonsäure-diarylester der allgemeinen Formel

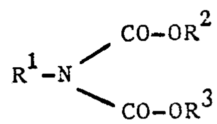

(I)

in welcher

$R^1$ für einen geradkettigen oder verzweigten Alkylrest mit 1-10 C-Atomen, der gegebenenfalls durch Niederalkoxy, Niederalkylmercapto, Halogen, Cyano oder Nitro substituiert sein kann ; einen Alkenylrest mit 3-8 C-Atomen ; einen Alkinylrest mit 3-8 C-Atomen ; einen cycloaliphatischen Rest mit 5-8 C-Atomen ; der gegebenenfalls durch Niederalkyl substituiert sein kann ; einen araliphatischen Rest mit 7-12 C-Atomen, wobei das aromatische Ringsystem gegebenenfalls durch Halogen, Nitro, Trifluormethyl, Cyano, Niederalkyl und/oder Niederalkoxy substituiert sein kann ; einen aromatischen Rest mit 6-12 C-Atomen, der gegebenenfalls durch Halogen, Nitro, Trifluormethyl, Cyano, Niederalkyl und/oder Niederalkoxy substituiert sein kann, oder für einen heterocyclischen Rest mit 5-6 Ringatomen, wobei 1-3 Heteroatome im Ringsystem vorhanden sein können, steht, und

$R^2$ und $R^3$ gleich oder verschieden sind und jeweils für einen gegebenenfalls durch Chlor, Methyl und/oder Methoxy substituierten Phenyl- oder Naphthylrest stehen,

erhält, wenn man Carbamidsäure-arylester der allgemeinen Formel

$$R^1—NH—CO—OR^2 \quad (II)$$

in welcher

$R^1$ und $R^2$ die oben angegebene Bedeutung haben,

mit Kohlensäure-arylester-halogeniden der allgemeinen Formel

$$X—CO—OR^3 \quad (III)$$

in welcher

$R^3$ die oben angegebene Bedeutung hat und

X für Halogen steht,

gegebenenfalls in Gegenwart eines Verdünnungsmittels, ohne Zusatz eines Säurebindemittels, bei Temperaturen zwischen 100 und 300 °C umsetzt.

Es ist als überraschend zu bezeichnen, daß die erfindungsgemäße Umsetzung abläuft, da Carbamidsäure-arylester mit Kohlensäure-arylester-halogeniden in Gegenwart eines säurebindenden Mittels nicht in gewünschter Weise reagieren : Es ist zwar bekannt — wie oben bereits erwähnt —, daß man N-substituierte Imido-dialkylester durch Umsetzung von N-substituierten Carbamidsäure-alkylestern mit Kohlensäure-alkylester-chloriden in Gegenwart von metallischem Natrium herstellen kann (vgl. J. Amer.

Chem. Soc. *69*, 2616-2618 (1947)).

Die Übertragung dieser Methode auf die entsprechenden Arylester versagt jedoch völlig. Beispielsweise reagiert Neopentyl-carbamidsäure-phenylester mit Kohlensäurephenylester-chlorid unter den in der zuletzt genannten Veröffentlichung beschriebenen Reaktionsbedingungen ausschließlich zu Neopentylisocyanat und Diphenylcarbonat. Auch mit Butyl-lithium als Säurefänger wird kein Neopentylimidodicarbonsäure-diphenylester gebildet. Umso überraschender ist es, daß bei erhöhter Temperatur und in Abwesenheit eines Säurefängers die erfindungsgemäße Reaktion glatt abläuft. Nach dem weiterhin bekannten Stand der Technik wäre zu erwarten gewesen, daß bei hoher Temperatur eine totale Rückspaltung der eingesetzten Carbamidsäure-arylester in Isocyanat und Phenol erfolgen würde (vgl. Houben-Weyl : Methoden der organischen Chemie, 4. Aufl. Bd. 8, S. 127 (1952)).

Verwendet man Neopentyl-carbamidsäure-phenylester und Kohlensäure-phenylester-chlorid als Ausgangsstoffe, so kann der Reaktionsverlauf durch das folgende Reaktionsschema wiedergegeben werden :

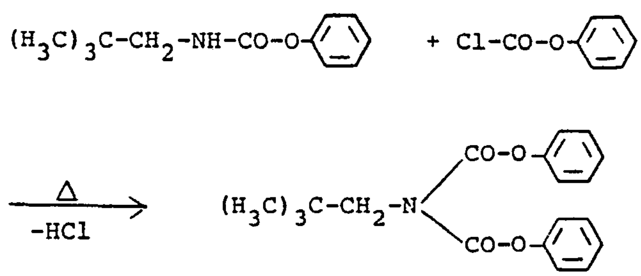


Die als Ausgangsstoffe zu verwendenden Carbamidsäurearylester sind durch die Formel (II) allgemein definiert.

$R^2$ steht in dieser Formel vorzugsweise für einen gegebenenfalls durch Chlor, Methyl und/oder Methoxy substituierten Phenyl- oder Naphthylrest. Die Ausdrücke « Niederalkyl », « Niederalkoxy » bzw. « Niederalkylmercapto » sollen im Rahmen dieser Erfindung entsprechende Reste mit jeweils 1-4 C-Atomen bedeuten.

Die erfindungsgemäß verwendbaren Carbamidsäure-arylester der Formel (II) sind bereits bekannt oder können nach bekannten Verfahren durch Addition von Isocyanaten an Phenole (vgl. Houben-Weyl, Methoden der organischen Chemie, 4. Aufl. Bd. 8, S. 142 (1952)) oder durch Umsetzung von Kohlensäurearylester-chloriden mit primären Aminen (vgl. Houben-Weyl, Methoden der organischen Chemie, 4. Aufl. Bd. 8, S. 138 (1952)) hergestellt werden. Die Ausgangsverbindungen der Formel (II) können ferner durch Umsetzung von Kohlensäurediarylestern mit Aminen hergestellt werden (vgl. Herstellungsbeispiel 5b ; vgl. auch Houben-Weyl, Methoden der organischen Chemie, 4. Aufl. Bd. 8, S. 139 (1952)).

Als Beispiele für Ausgangsverbindungen der Formel (II) seien im einzelnen genannt :

die Phenylester der Methyl-carbamidsäure, ferner der 2-Chlorethyl-, Isopropyl-, tert.-Butyl-, sec.-Butyl-, iso-Butyl-, tert.-Pentyl-, neo-Pentyl-, 1,2,2-Trimethylpropyl-, 2,2,2-Trifluorethyl-, 2-Ethoxyethyl-, 2-Ethyl-mercaptoethyl-, ω-Cyanohexyl-, Allyl-, Propargyl-, Cyclopentyl-, Cyclopropylmethyl-, Cyclopentylmethyl-, Cyclohexylmethyl-, (2,5-Methanocyclohexyl)-methyl-, Cycloheptylmethyl-, Cyclododecanylmethyl-, Adamantylmethyl-, 2-Furylmethyl-, 2-Pyranylmethyl-, 2-Pyridylmethyl-, 3-Pyridylmethyl-, 4-Pyridylmethyl-, 2-Methylpentyl-, 2-Ethylpentyl-, 2-Methylhexyl-, 2-Ethylhexyl-, 2-Methylcyclohexyl-, Benzyl-, 4-Chlorbenzyl-, 4-Nitrobenzyl-, Phenethyl-, Phenyl-, 4-Chlorphenyl-, 3,5-Dichlorphenyl-, 3,4-Dichlorpentyl-, 3-Trifluormethyl-, 2-Chlor-4-nitrophenyl-, 3-Tolyl-, 4-Ethylphenyl-, 3-Anisidyl-, 1-Naphthyl-, 2-Furyl-, 4-Pyridyl- und 2-Benzthiazolylcarbamidsäure.

Die weiterhin als Ausgangsstoffe zu verwendenden Kohlensäurearylester-halogenide sind durch die Formel (III) allgemein definiert. X in Formel (III) steht vorzugsweise für Chlor oder Fluor.

Die erfindungsgemäß verwendbaren Kohlensäure-arylesterhalogenide der Formel (III) sind bekannt oder können nach bekannten Verfahren hergestellt werden. So kann man z. B. die Kohlensäurephenylester-chloride in an sich bekannter Weise durch Phosgenierung von Phenolen herstellen (vgl. Houben-Weyl, Methoden der organischen Chemie, 4. Aufl., Bd. 8, S. 103 (1952)) ; die entsprechenden Kohlensäure-phenylester-fluoride können analog aus Phenolen und Difluorphosgen gewonnen werden (vgl. J. Chem. Soc. (London) *1948*, S. 2183).

Als Beispiele für Ausgangsverbindungen der Formel (III) seien im einzelnen genannt :

die Kohlensäureester-chloride des Phenols, 4-Chlorphenols, 4-Kresols oder 1-Naphthols sowie das Kohlensäureester-fluorid des Phenols.

Die erfindungsgemäße Umsetzung kann in Abwesenheit oder in Anwesenheit eines Verdünnungsmittels durchgeführt werden. Arbeitet man ohne Verdünnungsmittel, so wird der Carbamidsäurearylester (II) am zweckmäßigsten in geschmolzener Form zu dem im Reaktionsgefäß befindlichen, auf die Reaktionstemperatur erwärmten Kohlensäure-arylester-halogenid (III) zugegeben. Diese Ausführungsform ist in solchen Fällen möglich und besonders günstig, in denen der einzusetzende Carbamidsäurearylester (II) bei seiner Schmelztemperatur völlig stabil ist (vgl. Beispiel 5b).

Als Verdünnungsmittel für das Kohlensäure-arylester-halogenid (III), das im allgemeinen vorgelegt wird, kommen hochsiedende inerte organische Lösungsmittel, wie chlorierte oder nitrierte aromatische Kohlenwasserstoffe, z. B. Chlorbenzol, die Dichlorbenzole, die Trichlorbenzole oder Nitrobenzol, in Frage. Als Verdünnungsmittel für den zuzugebenden Carbamidsäure-arylester (II) können zweckmäßigerweise niedrig siedende inerte organische Lösungsmittel, wie Kohlenwasserstoffe und chlorierte Kohlenwasserstoffe, z. B. Petroläther, Cyclohexan, Dichlormethan, Chloroform oder Difluordichlormethan, vorzugsweise Dichlormethan, verwendet werden ; diese Lösungsmittel verdampfen bei der Reaktionstemperatur und dienen dadurch gleichzeitig als « Schleppmittel » zur Abführung des gebildeten Halogenwasserstoffs. Außerdem ist es möglich und in vielen Fällen besonders vorteilhaft, die Reaktion in einem Überschuß des als Reaktionskomponente dienenden Kohlensäure-arylester-halogenids (III) durchzuführen.

Das erfindungsgemäße Verfahren wird ohne Zusatz eines Säurebindemittels durchgeführt. Es hat sich jedoch als vorteilhaft erwiesen, den im Verlauf der Reaktion gebildeten Halogenwasserstoff rasch aus dem Reaktionsgemisch zu entfernen. Dies wird am zweckmäßigsten dadurch erreicht, daß man einen ständigen Luft- oder Stickstoffstrom durch das Reaktionsgemisch leitet, wenn man in einem hochsiedenden inerten organischen Lösungsmittel oder in überschüssigem Kohlensäurearylester-halogenid (III) als Verdünnungsmittel arbeitet. Bei Verwendung eines niedrigsiedenden Lösungsmittels, welches wie oben erwähnt als « Schleppmittel » zur Entfernung des gebildeten Halogenwasserstoffes fungiert, ist es dagegen nicht erforderlich, zusätzlich einen Luft- oder Stickstoffstrom durch das Reaktionsgemisch zu leiten.

Die Reaktionstemperaturen können in einem größeren Bereich variiert werden. Im allgemeinen arbeitet man, wie oben angegeben, zwischen etwa 100 und 300 °C, vorzugsweise zwischen etwa 170 und 250 °C.

Führt man die erfindungsgemäße Umsetzung in Gegenwart eines inerten organischen Lösungsmittels als Verdünnungsmittel durch, so setzt man auf 1 Mol eines Carbamidsäure-arylesters der Formel (II) im allgemeinen 1-15 Mol, vorzugsweise 4-12 Mol eines Kohlensäure-arylester-halogenids der Formel (III) ein. Verwendet man dagegen als Verdünnungsmittel einen Überschuß an Kohlensäure-arylester-halogenid (III), so kann man auf 1 Mol Carbamidsäure-arylester (II) bis zu 80 Mol, zweckmäßigerweise jedoch etwa 5-40 Mol, bevorzugt etwa 10-25 Mol Kohlensäure-arylester-halogenid (III) einsetzen. Es empfiehlt sich also in jedem Falle, das Kohlensäure-arylester-halogenid (III) in überstöchiometrischen Mengen einzusetzen.

Die Isolierung der Reaktionsprodukte erfolgt in einfacher Weise durch destillative Trennung des Reaktionsgemisches. Feste, höher schmelzende Imido-dicarbonsäure-diarylester lassen sich auch leicht durch Umkristallisieren reinigen.

Die erfindungsgemäß herstellbaren N-substituierten Imido-dicarbonsäure-diarylester der Formel (I), worin $R^1$ für Methyl steht, sind bekannt und können als Insektizide und Akarizide verwendet werden (DE-A-2 132 936). Die Verbindungen der Formel (I), worin $R^1$, $R^2$ und $R^3$ die oben angegebene Bedeutung haben — ausgenommen $R^1 = CH_3$ — sind neu. Bevorzugte neue N-substituierte Imido-dicarbonsäure-diarylester sind demnach solche Verbindungen der Formel (I), in welcher

$R^1$ für einen geradkettigen oder verzweigten Alkylrest mit 2-10 C-Atomen, der gegebenenfalls durch Niederalkoxy, Niederalkylmercapto, Halogen, Cyano oder Nitro substituiert sein kann ; einen Alkenylrest mit 3-8 C-Atomen ; einen Alkinylrest mit 3-8 C-Atomen ; einen cycloaliphatischen Rest mit 5-8 C-Atomen, der gegebenenfalls durch Niederalkyl substituiert sein kann ; einen araliphatischen Rest mit 7-12 C-Atomen, wobei das aromatische Ringsystem gegebenenfalls durch Halogen, Nitro, Trifluormethyl, Cyano, Niederalkyl und/oder Niederalkoxy substituiert sein kann ; einen aromatischen Rest mit 6-12 C-Atomen, der gegebenenfalls durch Halogen, Nitro, Trifluormethyl, Cyano, Niederalkyl und/oder Niederalkoxy substituiert sein kann, oder für einen heterocyclischen Rest mit 5-6 Ringatomen, wobei 1-3 Heteroatome im Ringsystem vorhanden sein können,
steht, und

$R^2$ und $R^3$ gleich oder verschieden sind und jeweils für einen gegebenenfalls durch Chlor, Methyl und/oder Methoxy substituierten Phenyl- oder Naphthylrest stehen.

Diese Verbindungen können als Zwischenprodukte zur Herstellung bekannter herbizider Wirkstoffe aus der Reihe der 1,3,5-Triazin-2,4 (1H,3H)-dione verwendet werden (vgl. z. B. DE-OS 2 254 200 und US-PS 4 056 527).

Besonders bevorzugte Verbindungen der allgemeinen Formel (I) sind die in den nachfolgenden Herstellungsbeispielen beschriebenen Verbindungen, da diese zu besonders wirksamen Herbizid-Endprodukten führen.

Nach einem bisher nicht zum Stand der Technik gehörenden Verfahren (welches Gegenstand eines gesonderten Schutzbegehrens ist ; vgl. EP-A2-0 034 751) kann man 1,3,5-Triazin-2,4-(1H,3H)-dione der allgemeinen Formel

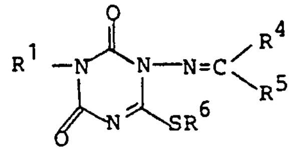

(IV)

in welcher

$R^1$ die oben angegebene Bedeutung hat und

$R^4$, $R^5$ und $R^6$ jeweils für gleiche oder verschiedene Alkylreste stehen,

mit hoher Ausbeute und Reinheit herstellen, wenn man die erfindungsgemäßen N-substituierten Imido-dicarbonsäure-diarylester der allgemeinen Formel

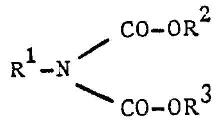

(I)

in welcher

$R^1$, $R^2$ und $R^3$ die oben angegebene Bedeutung haben,

mit einem Isothiosemicarbazon der allgemeinen Formel

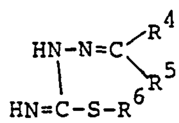

(V)

in welcher

$R^4$, $R^5$ und $R^6$ die oben angegebene Bedeutung haben, in etwa stöchiometrischen Mengen, ohne Verwendung eines Verdünnungsmittels und ohne Zusatz einer Base als Hilfsstoff, bei Temperaturen zwischen 50 und 150 °C, vorzugsweise zwischen 70 und 120 °C, umsetzt.

Die Aufarbeitung und Isolierung der Triazindione (IV) kann z. B. in der Weise erfolgen, daß man das bei der Kondensationsreaktion, (I) + (V) ⟶ (IV), gebildete (gegebenenfalls substituierte) Phenol bzw. Phenolgemisch im Vakuum abdestilliert und den Rückstand durch Destillation im Hochvakuum oder durch Umlösen, falls erforderlich, reinigt.

Die so hergestellten 1,3,5-Triazin-2,4 (1H,3H)-dione (IV) sind selbst herbizide Wirkstoffe ; sie können jedoch durch hydrolytische Abspaltung des als Schutzgruppe dienenden Alkylidenrestes (= $CR^4R^5$) auch leicht in die entsprechenden 1-Amino-1,3,5-triazin-2,4 (1H,3H-dione der allgemeinen Formel

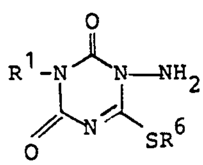

(VI)

in welcher

$R^1$ und $R^6$ die oben angegebene Bedeutung haben,

die ebenfalls ausgezeichnete Herbizide sind, umgewandelt werden. Ferner können die S-Alkylreste ($—SR^6$) in (IV) und (VI) durch Umsetzung mit primären oder sekundären Aminen gegen Alkylamino- bzw. Dialkylaminogruppen ausgetauscht werden, wobei ebenfalls bekannte herbizide Wirkstoffe erhalten werden (vgl. ebenfalls DE-OS 2 254 200 und US-PS 4 056 527).

Das hier angegebene neue, von den erfindungsgemäßen Imido-dicarbonsäure-diarylestern (I) ausgehende Herstellungsverfahren für die herbiziden Wirkstoffe der allgemeinen Formeln (IV) und (VI) sowie deren 6-Amino-derivate hat gegenüber den, z. B. aus DE-OS 2 254 200, vorbekannten Verfahren erhebliche und überraschende Vorteile. So kann man die Cyclisierungsreaktion ohne Verwendung von Lösungsmitteln in der Schmelze der Ausgangsprodukte durchführen. Hierbei werden keine weiteren Hilfsstoffe, wie z. B. organische Basen, benötigt. Einziges Nebenprodukt sind Phenole (keine Halogenwasserstoffe !), welche leicht abgetrennt und weiterverwendet werden können. Schließlich sind die als Ausgangsstoffe eingesetzten Imido-dicarbonsäure-diarylester (I) nach dem hier beanspruchten Verfahren auf technisch einfache Weise aus leicht zugänglichen Vorprodukten in hohen Ausbeuten herstellbar.

Die Isothiosemicarbazone der allgemeinen Formel (V) sind bekannt oder können nach bekannten Verfahren hergestellt werden, z. B. durch S-Alkylierung von Thiosemicarbazonen (vgl. Houben-Weyl, Methoden der organischen Chemie, 4. Auflage, Band 9, S. 912).

Nachfolgend wird beispielhaft die Synthese des besonders wirksamen herbiziden Wirkstoffes 1-Amino-6-ethylthio-3-neopentyl-1,3,5-triazin-2,4 (1H,3H)-dion (VIa) (vgl. z. B. DK-PS 136 067), ausgehend von der erfindungsgemäßen Verbindung N-Neopentyl-imido-dicarbonsäure-diphenylester (Ia) beschrieben ; der Reaktionsverlauf kann durch das folgende Formel-schema wiedergegeben werden :

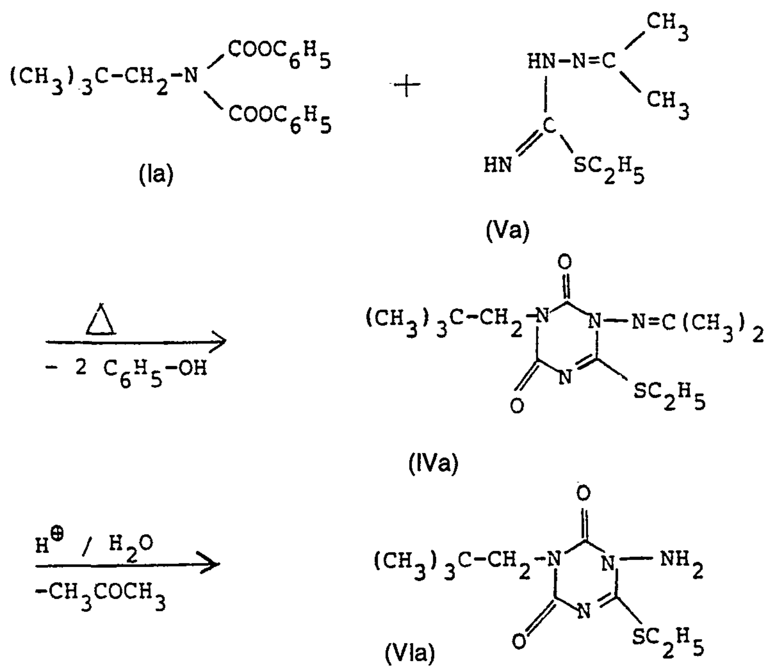


(Ia) (Va) (IVa) (VIa)

Eine Mischung von 65,4 g (0,2 Mol) N-Neopentyl-imido-dicarbonsäure-diphenylester (vgl. Herstellungsbeispiel 5) und 31,8 g (0,2 Mol) Aceton-S-ethyl-isothiosemicarbazon (Va) wird unter Stickstoff aufgeschmolzen und 5 Stunden bei 100 °C gerührt. Danach wird das gebildete Phenol im Vakuum abdestilliert. Der Rückstand, der im wesentlichen aus 1-Isopropyl-idenamino-6-ethylthio-3-neopentyl-1,3,5-triazin-2,4 (1H,3H)-dion (IVa) besteht, wird in 200 ml Isopropanol gelöst. Zur hydrolytischen Abspaltung der Isopropyliden-Schutzgruppe gibt man 2,8 g p-Toluolsulfonsäure zu und tropft bei einer Temperatur von 60 °C und einem Druck von 200-300 mbar innerhalb einer halben Stunde 14,4 ml Wasser zu. Das gebildete Aceton wird während der Reaktion zusammen mit etwa 100 ml Isopropanol abdestilliert. Das auskristallisierte 1-Amino-6-ethylthio-3-neopentyl-1,3,5-triazin-2,4 (1H,3H)-dion (VIa) wird bei 0 °C abgesaugt und mit Methanol gewaschen. Man erhält 38,2 g (VIa) vom Schmelzpunkt 202 °C, entsprechend einer Ausbeute von 74 % der Theorie.

In analoger Weise kann, ausgehend von der erfindungsgemäßen Verbindung N-Isobutyl-imido-dicarbonsäure-diphenylester (Ib), das bekannte, herbizid wirksame 1-Amino-3-isobutyl-6-methylthio-1,3,5-triazin-2,4 (1H,3H)-dion (VIb) (vgl. z. B. DK-PS 13 60 67) hergestellt werden, wobei das Zwischenprodukt 1-Isopropylidenamino-3-isobutyl-6-methylthio-1,3,5-triazin-2,4 (1H,3H)-dion (IVb) isoliert werden kann :

1. Stufe

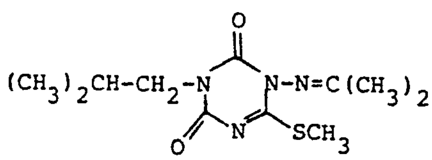

(IVb)

34,6 g (0,11 Mol) N-Isobutyl-imido-dicarbonsäure-diphenylester (Ib) (vgl. Herstellungsbeispiel 3) und 16,0 g (0,11 Mol) Aceton-S-methyl-isothiosemicarbazon werden bei 50 °C aufgeschmolzen und in einem Ölbad von 100 °C 4 Stunden lang gerührt. Man destilliert das gebildete Phenol bei einem Druck von 18 mbar ab, wobei man die Bad-Temperatur bis auf 140 °C steigert. Der Rückstand (30,3 g) erstarrt ; er wird mit 150 ml Cyclohexan aufgekocht, wobei 22,4 g reines 1-Isopropylidenamino-3-isobutyl-6-methylthio-1,3,5-triazin-2,4 (1H,3H)-dion (IVb) vom Schmelzpunkt 125-127 °C ungelöst zurückbleiben. Aus dem Filtrat davon kristallisieren weitere 6,4 g (IVb). Die Gesamtausbeute beträgt 28,8 g (97 % d. Th.). Die Verbindung (IVb) kann destilliert werden : Siedepunkt 165 °C bei 0,38 mbar.

2. Stufe

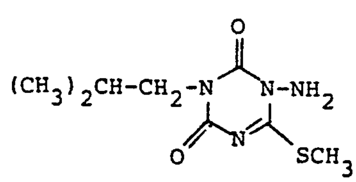

(VIb)

In einer Destillationsapparatur löst man 27,0 g (0,1 Mol) der Verbindung (IVb) bei 60 °C in 200 ml Isopropanol und stellt einen Druck von 260-200 mbar ein, so daß das Lösungsmittel zu sieden beginnt und im absteigenden Kühler kondensiert. Die Innentemperatur beträgt 45-50 °C. Sodann tropft man innerhalb 15 Minuten eine Lösung von 0,4 ml konzentrierter Schwefelsäure in 7 ml Wasser zu, wobei man während dieser Zeit ca. 70 ml Isopropanol, zusammen mit dem gebildeten Aceton, abdestilliert. Aus der verbleibenden Lösung kristallisieren bei 0 °C 14,5 g 1-Amino-3-isobutyl-6-methylthio-1,3,5-triazin-2,4 (1H, 3H)-dion (VIb) vom Schmelzpunkt 167-169 °C aus ; aus dem eingeengten Filtrat davon werden weitere 4,5 g erhalten. Die Gesamtausbeute von 19,0 g entspricht 83 % der Theorie.

Die nachfolgenden Herstellungsbeispiele sollen zur weiteren Erläuterung der Erfindung dienen.

## Beispiel 1

$$(CH_3)_2CH—N(CO—O—C_6H_5)_2 \quad (1)$$

In einem mit Rührer, Gaseinleitungsrohr, Rückflußkühler und Tropftrichter ausgerüsteten 4-Halskolben bringt man unter Durchleiten eines Luft- oder Stickstoff-Stroms 600 ml (4,72 Mol) Kohlensäurephenylester-chlorid zum Sieden. Bei einer Innentemperatur von 180-185 °C tropft man sodann innerhalb 5 Stunden eine Lösung von 71,6 g (0,4 Mol) Isopropyl-carbamidsäure-phenylester (Schmelzpunkt 78-80 °C) in 300 ml Kohlensäure-phenylester-chlorid (2,36 Mol) gleichmäßig zu, wobei weiter Luft oder Stickstoff durch die Reaktionslösung geleitet wird, um den gebildeten Chlorwasserstoff rasch abzuführen. Man rührt noch 2 Stunden bei Siede-Temperatur nach, destilliert das überschüssige Kohlensäurephenylester-chlorid bei einer Bad-Temperatur von 140 °C und einem Druck von 20 mbar ab und destilliert den Rückstand im Vakuum.

Man erhält 57,9 g N-Isopropyl-imido-dicarbonsäurediphenylester mit einem Siedepunkt von 155 °C/ 0,07 mbar und einem Schmelzpunkt von 35-37 °C. Die gaschromatografisch bestimmte Reinheit beträgt 98 % ; das entspricht einer Ausbeute von 47 % der Theorie.

## Beispiel 2

$$(CH_3)_3C—N(CO—O—C_6H_5)_2 \quad (2)$$

Man setzt, wie in Beispiel 1 beschrieben, 77,2 g (0,4 Mol) tert.-Butyl-carbamidsäure-phenylester (Fp. 92 °C) mit überschüssigem Kohlensäure-phenylester-chlorid (4,72 Mol) um und erhält nach dem Aufarbeiten 26,4 g N-tert.-Butyl-imido-dicarbonsäure-diphenylester mit einem Siedepunkt von 150 °C/ 0,1 mbar und einem Schmelzpunkt von 132 °C (aus Essigester).

Die gaschromatografisch bestimmte Reinheit beträgt 99,7 % ; das entspricht einer Ausbeute von 21 % der Theorie.

## Beispiel 3

$$(CH_3)_2CH—CH_2—N(CO—O—C_6H_5)_2 \quad (3)$$

Analog Beispiel 1 werden 77,2 g (0,4 Mol) Isobutyl-carbamidsäure-phenylester (Fp. 67 °C) mit überschüssigem Kohlensäure-phenylester-chlorid (4,72 Mol) umgesetzt. Man erhält nach der Destillation 102,4 g N-Isobutyl-imido-dicarbonsäure-diphenylester mit einem Siedepunkt von 160 °C/0,1 mbar und einer Reinheit von 93,7 %. Nach dem Umlösen aus ca. 500 ml Petrolether, Absaugen bei – 70 °C und Waschen mit tiefgekühltem Petroläther erhält man 88 g der genannten Verbindung mit einem Schmelzpunkt von 40 °C und einer Reinheit von 100 %, entsprechend 70 % der Theorie.

## Beispiel 4

$$C_2H_5—\underset{|}{\overset{CH_3}{C}}H—N(CO—O—C_6H_5)_2 \quad (4)$$

Analog Beispiel 1 setzt man 77,2 g (0,4 Mol) sec.-Butyl-carbamidsäure-phenylester (Fp. 43 °C) mit überschüssigem Kohlensäure-phenylester-chlorid (4,72 Mol) um. Nach dem Aufarbeiten erhält man 45,8 g N-sec.-Butyl-imido-dicarbonsäure-diphenylester mit einem Siedepunkt von 165-170 °C/0,2 mbar und vom Brechungsindex $n_D^{20} = 1,533\,6$. Die gaschromatografisch bestimmte Reinheit beträgt 97,8 %, entsprechend einer Ausbeute von 36 % der Theorie.

## Beispiel 5a

$$(CH_3)_3C—CH_2—N(CO—O—C_6H_5)_2 \quad (5)$$

Eine Lösung von 331 g (1,6 Mol) Neopentyl-carbamidsäure-phenylester in 1 000 g (6,39 Mol)

Kohlensäurephenylester-chlorid tropft man innerhalb von 5 Stunden in 4 000 g (25,56 Mol) siedendes Kohlensäure-phenylester-chlorid ein, durch das ein kräftiger Stickstoffstrom geleitet wird. Die Kopftemperatur am Rückflußkühler wird bei 80-90 °C gehalten, so daß das in geringer Menge als Nebenprodukt gebildete Neopentylisocyanat mit dem Stickstoff-Strom über Kopf destilliert und in einem nachgeschalteten absteigenden Kühler kondensiert werden kann. (Nach der Umsetzung mit Phenol zu Neopentyl-carbamidsäurephenylester wird es dann erneut der Reaktion zugeführt). Man rührt 4 Stunden unter weiterem Durchleiten von Stickstoff nach, destilliert sodann das überschüssige Kohlensäure-phenylester-chlorid bei einer Bad-Temperatur von 140 °C und einem Druck von 20 mbar ab und destilliert den Rückstand aus einem Heizbad von 170 °C, bis eine Siede-Temperatur von 150° bei einem Druck von 0,6 mbar erreicht ist. Der Rückstand besteht aus 96,5 %igem N-Neopentyl-imido-dicarbonsäure-diphenylester. Ausbeute : 489 g (90 % der Theorie). Eine aus Petrolether umkristallisierte Probe schmilzt bei 81 °C. Die Substanz ist destillierbar : Siedepunkt 156 °C/0,02 mbar.

Der noch nicht bekannte, als Ausgangsprodukt verwendete Neopentyl-carbamidsäure-phenylester kann beispielsweise, ausgehend von Neopentylamin, folgendermaßen hergestellt werden :

Eine Lösung von 80 g (2 Mol) Natriumhydroxid und 176 g (2 Mol) 99 %iges Neopentylamin in 3,4 Liter Wasser tropft man unter kräftigem Rühren in eine Lösung von 329 g (2,1 Mol) Kohlensäure-phenylester-chlorid in 1 Liter Toluol. Man hält durch Kühlung eine Innen-Temperatur von 10-20 °C. Nach beendeter Reaktion trennt man die Phasen, wäscht die organische Phase mit Wasser, filtriert und dampft zur Trockene ein. Man erhält 408 g eines 97 %igen Rohproduktes (95,6 % der Theorie) vom Schmelzpunkt 69-72 °C, für weitere Umsetzungen ausreichend rein. Nach dem Umlösen aus 2 Liter Petrolether erhält man 365 g vom Schmelzpunkt 77-78 °C.

Beispiel 5b

$$(CH_3)_3C—CH_2—N(CO—O—C_6H_5)_2 \quad (5)$$

Verfahrensvariante von Beispiel 5a

414 g (2 Mol) Neopentyl-carbamidsäure-phenylester werden als Schmelze aus einem auf 120 °C beheizten Tropftrichter innerhalb 8 Stunden gleichmäßig in 2,54 Liter (20 Mol) siedendes Kohlensäurephenylester-chlorid unter Durchleiten eines kräftigen Stickstoff-Stromes eingetropft. Man arbeitet analog Beispiel 5a auf und erhält 510 g eines 98 %igen N-Neopentyl-imido-dicarbonsäure-diphenylesters. Durch Aufarbeiten des zwischen 120 und 150 °C bei 0,6 mbar destillierten Anteils können weitere 37 g des gleichen Produktes gewonnen werden. Die Gesamtausbeute beträgt 82 % der Theorie N-Neopentyl-imido-dicarbonsäure-diphenylester.

Der als Ausgangsprodukt verwendete Neopentyl-carbamidsäure-phenylester wurde nach einem weiteren Verfahren wie folgt hergestellt :

2 140 g (10 Mol) Kohlensäure-diphenylester werden bei 80 °C aufgeschmolzen. Innerhalb 3 Stunden tropft man bei dieser Temperatur 870 g (10 Mol) Neopentylamin zu, destilliert das gebildete Phenol im Vakuum ab und kristallisiert den Rückstand aus Petroläther um. Man erhält 1 725 g (83 % der Theorie) Neopentyl-carbamidsäure-phenylester vom Schmelzpunkt 77-78 °C.

Beispiel 5c

$$(CH_3)_3C—CH_2—N(CO—O—C_6H_5)_2 \quad (5)$$

Verfahrensvariante von Beispielen 5a und 5b

Ein 4-Liter-Dreihalskolben wird mit Rührer und Rückflußkühler ausgerüstet. Auf dem Rückflußkühler befindet sich ein Reitmeir-Aufsatz, danach eine absteigende Destillationsbrücke mit einem Intensivkühler. In den Rührkolben taucht eine spezielle Gaseinleitungsfritte, auf der über eine Schliffverbindung ein Tropftrichter angebracht ist. Das Gas-Zuführungsrohr der Fritte wird zum Druckausgleich mit dem Tropftrichter verbunden. Der Rückflußkühler wird mit Wasser von 80-95 °C beschickt, der absteigende Kühler mit kaltem Wasser.

Im Rührkolben werden sodann 2,54 Liter (20 Mol) Kohlensäure-phenylesterchlorid zum Sieden erhitzt. Bei einer Sumpf-Temperatur von 185-190 °C tropft man sodann eine Lösung von 414 g (2 Mol) Neopentyl-carbamidsäure-phenylester in 300 ml Dichlormethan innerhalb 9 Stunden gleichmäßig in die Gaseinleitungsfritte, wobei das verdampfende Lösungsmittel den gebildeten Chlorwasserstoff sofort aus dem Reaktionsmedium abführt. Das Lösungsmittel wird im Intensivkühler kondensiert, der mitgeführte Chlorwasserstoff in einem nachgeschalteten Berieselungsturm in Wasser absorbiert. Das siedende Kohlensäure-phenylester-chlorid wird auf diese Weise im Rückflußkühler kondensiert, und ein Mitreißen mit dem Dichlormethan-Dampf wird durch den Reitmeier-Aufsatz verhindert.

Man arbeitet analog Beispiel 5a auf und erhält insgesamt 581 g eines 98 %igen N-Neopentyl-imido-dicarbonsäure-diphenylesters, entsprechend einer Ausbeute von 87 % der Theorie.

Beispiel 6

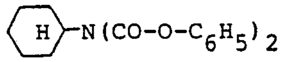

(6)

Eine Lösung von 87,6 g (0,4 Mol) Cyclohexylcarbamidsäure-phenylester (Fp. 136-137 °C) in 1 000 g (6,4 Mol) Kohlensäure-phenylester-chlorid tropft man in 750 g (4,8 Mol) siedendes Kohlensäure-phenylester-chlorid ein, kocht noch 3 Stunden unter Durchleiten von Stickstoff unter Rückfluß und arbeitet analog Beispiel 1 auf.

Man erhält 83,1 g (N-Cyclohexyl-imido-dicarbonsäure-diphenylester mit einem Siedebreich von 165-175 °C bei 0,1 mbar und einer gas-chromatografisch bestimmten Reinheit von 97,5 %. Eine aus Petrolether umkristallisierte Probe schmilzt bei 85 °C. Ausbeute : 60 % der Theorie.

Beispiel 7

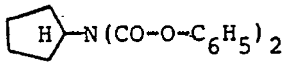

(7)

Analog Beispiel 6 werden 83,0 g (0,4 Mol) Cyclopentylcarbamidsäure-phenylester (Fp. 115-117 °C) mit überschüssigem Kohlensäure-phenylester-chlorid (insgesamt 8,8 Mol) umgesetzt. Man erhält 81,2 g N-Cyclopentyl-imido-dicarbonsäure-diphenylester mit einem Siedebereich von 155-182 °C bei 0,1 mbar und einer gaschromatografisch bestimmten Reinheit von 86,8 %, entsprechend einer Ausbeute von 54,2 % der Theorie. Nach dem Umlösen aus 300 ml Petrolether erhält man 52 g vom Schmelzpunkt 53 °C.

Beispiel 8

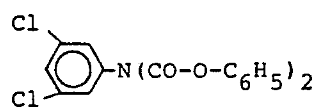

(8)

Eine Lösung von 56,4 g (0,2 Mol) 3,5-Dichlorphenyl-carbamidsäure-phenylester (Fp. 141 °C) in 1 015 ml (8,0 Mol) Kohlensäure-phenylester-chlorid tropft man innerhalb 5 Stunden in 306 ml (2,4 Mol) siedendes Kohlensäurephenylester-chlorid ein. Dabei wird Stickstoff durch die Reaktionslösung geleitet. Danach kocht man weitere 4 Stunden unter Rückfluß, destilliert das überschüssige Kohlensäure-phenylester-chlorid bei einer Bad-Temperatur von 140 °C und einem Druck von 20 mbar ab. Der Rückstand enthält als Verunreinigungen 3,5-Dichlorphenylisocyanat und Diphenylcarbonat.

Diese werden bei einer Bad-Temperatur von 150 °C und einem Druck von 0,3 mbar weitgehend abdestilliert. Der Destillationsrückstand wird aus 300 ml Essigsäureethylester umkristallisiert. Man erhält 15,6 g (19,4 % der Theorie) N-(3,5-Dichlorphenyl)-imido-dicarbonsäure-diphenylester vom Schmelzpunkt 178-180 °C.

Beispiel 9

$$(C_2H_5)_2CH—N(CO—O—C_6H_5)_2 \qquad (9)$$

Eine Lösung von 82,8 g (0,4 Mol) (1-Ethyl-propyl)-carbamidsäure-phenylester (Fp. 65-67 °C) in 500 ml (3,9 Mol) Kohlensäure-phenylester-chlorid tropft man innerhalb 5 Stunden in 600 ml (4,7 Mol) siedendes Kohlensäure-phenylester-chlorid ein und leitet dabei einen kräftigen Stickstoff-Strom durch die siedende Lösung. Man kocht noch 4 Stunden unter Rückfluß, destilliert das überschüssige Kohlensäure-phenylester-chlorid im Vakuum ab und destilliert den Rückstand im Hochvakuum.

Man erhält 84,6 g (64,7 %) N-(1-Ethyl-propyl)-imido-dicarbonsäure-diphenylester mit einem Siedepunkt von 134 °C/0,09 mbar und einem Schmelzpunkt von 54-56 °C (aus Pentan).

Beispiel 10

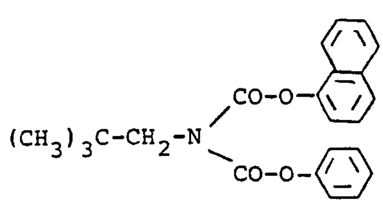

(10)

Eine Lösung von 59,1 g (0,23 Mol) Neopentyl-carbamidsäure-α-naphthylester (Fp. 116-117 °C) in 600 ml (4,7 Mol) Kohlensäure-phenylester-chlorid läßt man innerhalb von 5 Stunden in 300 ml (2,35 Mol)

siedendes Kohlensäure-phenylester-chlorid eintropfen, wobei ein kräftiger Stickstoffstrom durch die Lösung geleitet wird. Nach dem Abdestillieren des überschüssigen Kohlensäure-phenylester-chlorids destilliert man den verbleibenden Rückstand (83,0 g, nach Gaschromatogramm 95,4 %ig, entsprechend einer Ausbeute von 91,3 %) im Hochvakuum.

Man erhält 52,7 g N-Neopentyl-imido-dicarbonsäurephenylester-α-naphthylester vom Siedepunkt 230-235 °C/0,25 mbar.

Beispiel 11

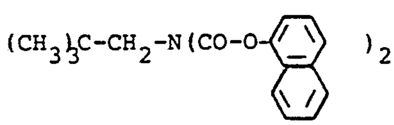

(11)

Eine Lösung von 29,4 g (0,114 Mol) Neopentyl-carbamidsäure-α-naphthylester (Fp. 117-118 °C) in 510 g (2,47 Mol) Kohlensäure-α-naphthylester-chlorid tropft man innerhalb 5 Stunden in 250 g (1,21 Mol) auf 230-240 °C erhitztes Kohlensäure-α-naphthylester-chlorid ein, wobei ein kräftiger Luftstrom durch die Lösung geleitet wird. Man rührt noch 4 Stunden bei 240 °C unter weiterem Durchleiten von Luft, destilliert den Überschuß an Kohlensäure-α-naphthylester (Kp. 155 °C bei 20 mbar) ab und entfernt anschließend die noch vorhandenen Reste durch Erhitzen auf 140 °C bei 0,03 mbar.

Der Rückstand (49 g) wird zweimal aus jeweils 500 ml Petrolether umkristallisiert.

Man erhält 22,9 g (47 %) N-Neopentyl-imido-dicarbonsäure-di-α-naphthylester vom Schmelzpunkt 92-93 °C.

Beispiel 12

$$CF_3—CH_2—N(CO—O—C_6H_5)_2 \qquad (12)$$

Man trägt 21,9 g (0,1 Mol) 2,2,2-Trifluorethyl-carbamidsäure-phenylester (Fp. 88-90 °C) in 85 ml (0,67 Mol) Kohlensäure-phenylester-chlorid ein und erhitzt 20 Stunden unter Rückfluß. Nach Abdestillation des überschüssigen Kohlensäure-phenylester-chlorides wird der Rückstand über eine kurze Vigreux-Kolonne bis zum Erreichen eines Siedepunktes von 132 °C bei 0,1 mbar andestilliert. Man erhält als Rückstand 28,7 g Rohprodukt, das gemäß Gaschromatogramm aus 98 %igem N-(2,2,2-Trifluorethyl)-imido-dicarbonsäure-diphenyl-ester besteht, (entsprechend einer Ausbeute von 83 % der Theorie). Eine aus Waschbenzin umkristallisierte Probe schmilzt bei 76 °C. Der Siedepunkt beträgt 140 °C bei 0,3 mbar.

**Ansprüche**

1. Verfahren zur Herstellung von N-substituierten Imido-dicarbonsäure-diarylestern der allgemeinen Formel (I)

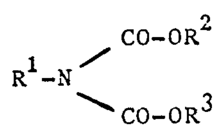

(I)

in welcher

$R^1$ für einen geradkettigen oder verzweigten Alkylrest mit 1-10 C-Atomen, der gegebenenfalls durch Niederalkoxy, Niederalkylmercapto, Halogen, Cyano oder Nitro substituiert sein kann ; einen Alkenylrest mit 3-8 C-Atomen ; einen Alkinylrest mit 3-8 C-Atomen ; einen cycloaliphatischen Rest mit 5-8 C-Atomen ; der gegebenenfalls durch Niederalkyl substituiert sein kann ; einen araliphatischen Rest mit 7-12 C-Atomen, wobei das aromatische Ringsystem gegebenenfalls durch Halogen, Nitro, Trifluormethyl, Cyano, Niederalkyl und/oder Niederalkoxy substituiert sein kann ; einen aromatischen Rest mit 6-12 C-Atomen, der gegebenenfalls durch Halogen, Nitro, Trifluormethyl, Cyano, Niederalkyl und/oder Niederalkoxy substituiert sein kann, oder für einen heterocyclischen Rest mit 5-6 Ringatomen, wobei 1-3 Heteroatome im Ringsystem vorhanden sein können, steht, und

$R^2$ und $R^3$ gleich oder verschieden sind und jeweils für einen gegebenenfalls durch Chlor, Methyl und/oder Methoxy substituierten Phenyl- oder Naphthylrest stehen,

dadurch gekennzeichnet, daß man Carbamidsäure-arylester der allgemeinen Formel

$$R^1—NH—CO—OR^2 \qquad (II)$$

in welcher

$R^1$ und $R^2$ die oben angegebene Bedeutungen haben,

mit Kohlensäure-arylester-halogeniden der allgemeinen Formel

$X-CO-OR^3$ (III)

in welcher

$R^3$ die oben angegebene Bedeutung hat und

X für Halogen steht,

gegebenenfalls in Gegenwart eines Verdünngsmittels, ohne Zusatz eines Säurebindemittels, bei Temperaturen zwischen 100 und 300 °C umsetzt.

2. Verfahren nach Anspruch 1, dadurch gekennzeichnet, daß man die Umsetzung bei Temperaturen zwischen 170 und 250 °C durchführt.

3. Verfahren nach Anspruch 1, dadurch gekennzeichnet, daß man das Kohlensäure-arylester-halogenid (III) in überstöchiometrischen Mengen einsetzt.

4. Verfahren nach Anspruch 1, dadurch gekennzeichnet, daß man Kohlensäure-arylester-halogenide der Formel (III) einsetzt, in welcher

X für Chlor oder Fluor steht.

5. N-substituierte Imido-dicarbonsäure-diarylester der allgemeinen Formel

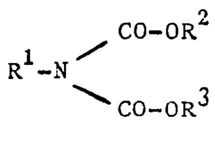
(I)

in welcher

$R^1$ für einen geradkettigen oder verzweigten Alkylrest mit 2-10 C-Atomen, der gegebenenfalls durch Niederalkoxy, Niederalkylmercapto, Halogen, Cyano oder Nitro substituiert sein kann ; einen Alkenylrest mit 3-8 C-Atomen ; einen Alkinylrest mit 3-8 C-Atomen ; einen cycloaliphatischen Rest mit 5-8 C-Atomen, der gegebenenfalls durch Niederalkyl substituiert sein kann ; einen araliphatischen Rest mit 7-12 C-Atomen, wobei das aromatische Ringsystem gegebenenfalls durch Halogen, Nitro, Trifluormethyl, Cyano, Niederalkyl und/oder Niederalkoxy substituiert sein kann ; einen aromatischen Rest mit 6-12 C-Atomen, der gegebenenfalls durch Halogen, Nitro, Trifluormethyl, Cyano, Niederalkyl und/oder Niederalkoxy substituiert sein kann, oder für einen heterocyclischen Rest mit 5-6 Ringatomen, wobei 1-3 Heteroatome im Ringsystem vorhanden sein können, steht, und

$R^2$ und $R^3$ gleich oder verschieden sind und jeweils für einen gegebenenfalls durch Chlor, Methyl und/oder Methoxy substituierten Phenyl- oder Naphthylrest stehen.

6. N-Isopropyl-imido-dicarbonsäure-diphenylester,
N-tert.-Butyl-imido-dicarbonsäure-diphenylester,
N-Isobutyl-imido-dicarbonsäure-diphenylester,
N-sec.-Butyl-imido-dicarbonsäure-diphenylester,
N-Neopentyl-imido-dicarbonsäure-diphenylester,
N-Cyclohexyl-imido-dicarbonsäure-diphenylester,
N-Cyclopentyl-imido-dicarbonsäure-diphenylester,
N-(3,5-Dichlorphenyl)-imido-dicarbonsäure-diphenylester,
N-(1-Ethyl-propyl)-imido-dicarbonsäure-diphenylester,
N-Neopentyl-imido-dicarbonsäure-phenylester-α-naphthylester,
N-Neopentyl-imido-dicarbonsäure-di-α-naphthylester
und
N-(2,2,2-Trifluorethyl)-imido-dicarbonsäure-diphenylester
gemäß Anspruch 5.

**Claims**

1. Process for the preparation of N-substituted imido-dicarboxylic acid diaryl esters of the general formula (I)

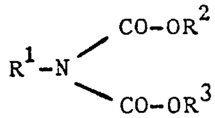
(I)

in which

$R^1$ represents a straight-chain or branched alkyl radical which has 1-10 C atoms and can optionally be substituted by lower alkoxy, lower alkylmercapto, halogen, cyano or nitro ; an alkenyl radical with 3-8 C atoms ; an alkinyl radical with 3-8 C atoms ; a cycloaliphatic radical with 5-8 C atoms which can optionally be substituted by lower alkyl ; an araliphatic radical with 7-12 C atoms, it being possible for the aromatic ring system to be optionally substituted by halogen, nitro, trifluoromethyl, cyano, lower alkyl and/or lower alkoxy ; an aromatic radical which has 6-12 C atoms and can optionally be substituted by halogen, nitro, trifluoromethyl, cyano, lower alkyl and/or lower alkoxy, or a heterocyclic radical with 5-6

ring atoms, it being possible for 1-3 hetero atoms to be present in the ring system, and

$R^2$ and $R^3$ are identical or different and each represent a phenyl or naphthyl radical which is optionally substituted by chlorine, methyl and/or methoxy,

characterised in that carbamic acid aryl esters of the general formula

$$R^1—NH—CO—OR^2 \quad (II)$$

in which

$R^1$ and $R^2$ have the meanings indicated above,

are reacted with carbonic acid aryl ester halides of the general formula

$$X—CO—OR^3 \quad (III)$$

in which

$R^3$ has the meaning indicated above and

X represents halogen,

if appropriate in the presence of a diluent, without the addition of an acid-binding agent, at temperatures between 100 and 300 °C.

2. Process according to Claim 1, characterised in that the reaction is carried out at temperatures between 170 and 250 °C.

3. Process according to Claim 1, characterised in that the carbonic acid aryl ester halide (III) is used in amounts which are greater than the stoichiometric amount.

4. Process according to Claim 1, characterised in that carbonic acid aryl ester halides of the formula (III) in which

X represents chlorine or fluorine,

are used.

5. N-substituted imido-dicarboxylic acid diaryl esters of the general formula

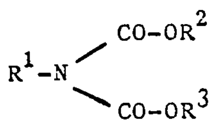

(I)

in which

$R^1$ represents a straight-chain or branched alkyl radical which has 2-10 C atoms and can optionally be substituted by lower alkoxy, lower alkylmercapto, halogen, cyano or nitro ; an alkenyl radical with 3-8 C atoms ; an alkinyl radical with 3-8 C atoms ; a cycloaliphatic radical which has 5-8 C atoms and can optionally be substituted by lower alkyl ; an araliphatic radical with 7-12 C atoms, it being possible for the aromatic ring system to be optionally substituted by halogen, nitro, trifluoromethyl, cyano, lower alkyl and/or lower alkoxy ; an aromatic radical which has 6-12 C atoms and can optionally be substituted by halogen, nitro, trifluoromethyl, cyano, lower alkyl and/or lower alkoxy, or a heterocyclic radical with 5-6 ring atoms, it being possible for 1-3 hetero atoms to be present in the ring system, and

$R^2$ and $R^3$ are identical or different and each represent a phenyl or naphthyl radical which is optionally substituted by chlorine, methyl and/or methoxy.

6. N-isopropyl-imido-dicarboxylic acid diphenyl ester,
N-tert.-butyl-imido-dicarboxylic acid diphenyl ester,
N-isobutyl-imido-dicarboxylic acid diphenyl ester,
N-sec.-butyl-imido-dicarboxylic acid diphenyl ester,
N-neopentyl-imido-dicarboxylic acid diphenyl ester,
N-cyclohexyl-imido-dicarboxylic acid diphenyl ester,
N-Cyclopentyl-imido-dicarboxylic acid diphenyl ester,
N-(3,5-dichlorophenyl)-imido-dicarboxylic acid diphenyl ester,
N-(1-ethyl-propyl)-imido-dicarboxylic acid diphenyl ester,
N-neopentyl-imido-dicarboxylic acid phenyl ester α-naphthyl ester,
N-neopentyl-imido-dicarboxylic acid di-α-naphthyl ester
and
N-(2,2,2-trifluoroethyl)-imido-dicarboxylic acid diphenyl ester,
according to Claim 5.

**Revendications**

1. Procédé de préparation d'esters diaryliques d'acides imido-dicarboxyliques N-substitués de formule générale (I)

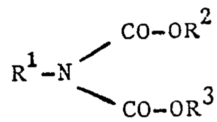

dans laquelle

$R^1$ représente un groupe alkyle à chaîne droite ou ramifiée contenant 1 à 10 atomes de carbone et pouvant être éventuellement substitué par un groupe alcoxy inférieur, par un groupe alkyl inférieur-mercapto, par un atome d'halogène, par un groupe cyano ou par un groupe nitro ; un groupe alcényle contenant 3 à 8 atomes de carbone ; un groupe alcinyle contenant 3 à 8 atomes de carbone ; un radical cycloaliphatique contenant 5 à 8 atomes de carbone et pouvant être éventuellement substitué par un groupe alkyle inférieur ; un radical araliphatique contenant 7 à 12 atomes de carbone, le système à noyau aromatique pouvant éventuellement être substitué par un atome d'halogène, par un groupe nitro, par un groupe trifluorométhyle, par un groupe cyano, par un groupe alkyle inférieur et/ou par un groupe alcoxy inférieur ; un radical aromatique contenant 6 à 12 atomes de carbone et pouvant éventuellement être substitué par un atome d'halogène, par un groupe nitro, par un groupe trifluorométhyle, par un groupe cyano, par un groupe alkyle inférieur et/ou par un groupe alcoxy inférieur, ou un radical hétérocyclique contenant 5 à 6 atomes cycliques, 1-3 hétéro-atomes pouvant être présents dans le système cyclique, et

$R^2$ et $R^3$ sont identiques ou différents et représentent chacun un groupe phényle ou un groupe naphtyle éventuellement substitué par un atome de chlore, par un groupe méthyle et/ou par un groupe méthoxy,

caractérisé en ce qu'on fait réagir des esters aryliques d'acide carbamique de formule générale

$$R^1\text{—NH—CO—OR}^2 \qquad \text{(II)}$$

dans laquelle

$R^1$ et $R^2$ ont les significations indiquées ci-dessus, avec des halogénures d'esters aryliques d'acide carbonique de formule générale

$$X\text{—CO—OR}^3 \qquad \text{(III)}$$

dans laquelle

$R^3$ a la signification indiquée ci-dessus, et

X représente un atome d'halogène,

éventuellement en présence d'un diluant, sans ajouter un agent fixateur d'acide, à des températures comprises entre 100 et 300 °C.

2. Procédé suivant la revendication 1, caractérisé en ce qu'on effectue la réaction à des températures comprises entre 170 et 250 °C.

3. Procédé suivant la revendication 1, caractérisé en ce qu'on utilise l'halogénure d'ester arylique d'acide carbonique (III) en quantités hyperstœchiométriques.

4. Procédé suivant la revendication 1, caractérisé en ce qu'on utilise des halogénures d'esters aryliques d'acide carbonique de formule (III) dans laquelle

X représente un atome de chlore ou un atome de fluor.

5. Esters diaryliques d'acides imido-dicarboxyliques N-substitués de formule générale

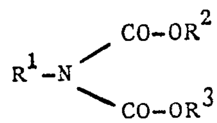

(I)

dans laquelle

$R^1$ représente un groupe alkyle à chaîne droite ou ramifiée contenant 2 à 10 atomes de carbone et pouvant éventuellement être substitué par un groupe alcoxy inférieur, par un groupe alkyl inférieur-mercapto, par un atome d'halogène, par un groupe cyano ou par un groupe nitro ; un groupe alcényle contenant 3 à 8 atomes de carbone ; un groupe alcinyle contenant 3 à 8 atomes de carbone ; un radical cycloaliphatique contenant 5 à 8 atomes de carbone et pouvant éventuellement être substitué par un groupe alkyle inférieur ; un radical araliphatique contenant 7 à 12 atomes de carbone, le système à noyau aromatique pouvant éventuellement être substitué par un atome d'halogène, par un groupe nitro, par un groupe trifluorométhyle, par un groupe cyano, par un groupe alkyle inférieur et/ou par un groupe alcoxy inférieur ; un radical aromatique contenant 6 à 12 atomes de carbone et pouvant éventuellement être substitué par un atome d'halogène, par un groupe nitro, par un groupe trifluorométhyle, par un groupe cyano, par un groupe alkyle inférieur et/ou par un groupe alcoxy inférieur, ou un radical hétérocyclique contenant 5 ou 6 atomes cycliques, 1-3 hétéroatomes pouvant être présents dans le système cyclique, et

$R^2$ et $R^3$ sont identiques ou différents et représentent chacun un groupe phényle ou un groupe naphtyle éventuellement substitué par un atome de chlore, par un groupe méthyle et/ou par un groupe méthoxy.

6. L'ester diphénylique d'acide N-isopropyl-imido-dicarboxylique,
l'ester diphénylique d'acide N-tert-butyl-imido-dicarboxylique,
l'ester diphénylique d'acide N-isobutyl-imido-dicarboxylique,
l'ester diphénylique d'acide N-sec-butyl-imido-dicarboxylique,
l'ester diphénylique d'acide N-néopentyl-imido-dicarboxylique,
l'ester diphénylique d'acide N-cyclohexyl-imido-dicarboxylique,
l'ester diphénylique d'acide N-cyclopentyl-imido-dicarboxylique,
l'ester diphénylique d'acide N-(3,5-dichlorophényl)-imido-dicarboxylique,
l'ester diphénylique d'acide N-(1-éthyl-propyl)-imido-dicarboxylique,
l'ester α-naphtylique de l'ester phénylique d'acide N-néopentyl-imido-dicarboxylique,
l'ester di-α-naphtylique d'acide N-néopentyl-imido-dicarboxylique,
et
l'ester diphénylique d'acide N-(2,2,2-trifluoréthyl)-imido-dicarboxylique
suivant la revendication 5.